# FASCICULE DE BREVET EUROPEEN

(11) **EP 3 551 239 B1**
(45) Date de publication et mention de la délivrance du brevet: **13.07.2022**
(21) Numéro de dépôt: 17816977.7
(22) Date de dépôt: 04.12.2017
(51) Int. Cl.: A61L 9/014, E04F 13/00, E04B 9/30

(54) **STRUCTURE DE NEUTRALISATION D'ODEURS**
GERUCHSNEUTRALISATIONSSTRUKTUR
ODOR NEUTRALIZATION STRUCTURE

(30) Priorité: 06.12.2016 FR 1661976
(43) Date de publication de la demande: 16.10.2019
(73) Titulaire: Normalu, 68680 Kembs (FR)
(72) Inventeur: SCHERRER, Jean-Marc, 68400 Riedishiem (FR)
(74) Mandataire: IP Trust
(86) Numéro de dépôt international: PCT/FR2017/053367
(87) Numéro de publication internationale: WO 2018/104636

(56) Documents cités:
- EP-A1- 2 977 518
- WO-A1-2004/058313
- JP-A- 2006 299 713

## Description

L'invention concerne le traitement d'odeurs qualifiées de « mauvaises » présentes dans un environnement clos (local).

L'invention concerne plus particulièrement une structure de neutralisation d'odeurs ambiantes qualifiées de mauvaises odeurs. Dans la présente demande, le terme « neutralisation » englobe également les termes de « réduction », « limitation « et « suppression » d'odeurs.

Différentes causes peuvent être à l'origine de mauvaises odeurs dans un local. Dans un lieu d'habitation, il peut s'agir par exemple de tabac, d'aliments, de bactéries, moisissures etc. Les mauvaises odeurs dans un local sont traitées habituellement selon leur cause, soit en supprimant la source même de la mauvaise odeur, soit en masquant cette dernière par diffusion d'un agent de masquage, soit en la diminuant au moyen d'une substance adaptée pour absorber/adsorber la mauvaise odeur.

S'agissant du masquage ou l'absorption / adsorption d'odeurs, il est répandu d'utiliser des diffuseurs de substances parfumées ou des récipients contenant des compositions ayant des propriétés d'absorption / d'adsorption d'odeurs. Par exemple, il est connu de JP 2006 299713 A de disposer un déodorant derrière une fausse paroi. L'inconvénient des diffuseurs et récipients d'absorption / adsorption est leur support. Ces derniers sont disposés classiquement sur des tables, meubles ou équivalents. Selon la destination du local, il peut d'avérer que de tels supports ne soient pas toujours présents. Par ailleurs, la présence visuelle de tels produits peut s'avérer dans certain cas non agréable voire non appropriée.

L'invention vise à remédier à ces problèmes en proposant une structure de neutralisation d'odeur(s) disposée dans un local sans interférer l'aspect visuel de celui-ci et ce indépendamment de tout arrangement ou mobilier du local.

### OBJET DE L'INVENTION

A cet effet, et selon un premier aspect, l'invention propose une structure de neutralisation d'odeur(s) selon la revendication 1 comprenant un profilé de lisse adapté pour permettre l'accroche sur celui-ci d'une toile tendue pour la réalisation d'une fausse-paroi et au moins une cartouche filtrante contenant un matériau sorbant d'émanation volatiles (odeurs) spécifiques, ladite cartouche étant logée dans une gorge du profilé de lisse. Les structures comportant un profilé de lisse adapté pour permettre l'accrochage d'une feuille tendue et comportant des gorges sont en tant que telles connues, par exemple du document EP 2 977 518 A1.

En équipant des profilés de lisse d'une ou plusieurs cartouche(s) filtrante(s) comme décrit ci-dessus, il est possible d'installer des fausses paroi (faux mur, faux plafond, parois décoratives) ayant la fonction, en plus de leur fonction première de masquage d'éléments techniques ou de décoration, de neutraliser les odeurs sur l'ensemble du local. Ainsi, quelle que soit la taille du local et quel que soit l'arrangement intérieur du local, la neutralisation d'odeurs est assurée sans que l'aspect visuel du local ne soit altéré, les cartouches filtrantes étant invisibles depuis le local.

Avantageusement, le matériau sorbant comprend de la zéolithe.

Avantageusement, la cartouche filtrante contient en outre une substance parfumée, ladite cartouche étant arrangée pour libérer ladite substance.

Avantageusement, la gorge présente une ouverture débouchant au-dessus de la face interne de la toile lorsque cette dernière est fixée sur le profilé de lisse. On entend par face interne de la toile, la face de la toile orientée du côté opposé à l'intérieur du local lorsque la toile est accrochée sur le profilé de lisse fixée à une paroi du local. Par opposition, la face externe de la toile désigne la face orientée vers l'intérieur du local.

Avantageusement, la gorge présente une ouverture débouchant en partie inférieure du profilé de lisse. Cette configuration a pour avantage de faciliter l'accès à la gorge contenant la cartouche filtrante et ainsi faciliter la pose, le retrait ou le remplacement de la cartouche.

Avantageusement, la gorge présente une ouverture débouchant dans un espace délimité par la face interne de ladite toile lorsque cette dernière est fixée sur le profilé de lisse.

Avantageusement, le profilé de lisse comporte un clapet d'accès à la gorge

Avantageusement, la gorge est fermée par un cache pourvu de trous pour le passage de composés volatiles.

L'invention concerne également une fausse paroi comprenant une toile tendue sur une lisse formée par au moins un profilé de lisse constituant une structure de neutralisation d'odeur(s) telle que décrite précédemment.

Selon une configuration particulière, la toile est micro-perforée. Cela a pour avantage de permettre la neutralisation des odeurs et/ou le passage de la substance parfumée également dans l'espace délimité par la face interne de la toile.

Avantageusement, la gorge dans laquelle la cartouche filtrante est fixée est une gorge accessible depuis le local dans lequel la fausse-paroi est montée. Cela rend ainsi la cartouche filtrante accessible depuis le local sans devoir retirer la toile.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique en coupe d'un profilé de lisse formant une structure de neutralisation d'odeurs selon un premier exemple de réalisation de l'invention, une toile tendue étant montrée accrochée sur ledit profilé ;
- la figure 2 représente une variante de réalisation du profilé de lisse de la figure 1 ;
- La figure 3 représente une variante de réalisation de profilé de lisse de la figure 2 ;
- la figure 4 représente une vue schématique en coupe d'un profilé de lisse formant une structure de neutralisation d'odeurs selon un deuxième exemple de réalisation de l'invention, une toile tendue étant montrée accrochée sur ledit profilé ;
- les figures 5a et 5b montre une variante de réalisation du profilé de lisse de la figure 4.

Pour plus de clarté, les éléments identiques ou similaires des différents modes de réalisation sont repérés par des signes de référence identiques sur l'ensemble des figures.

### DESCRIPTION DETAILLEE DES FIGURES

Les profilés de lisse illustrés sur les figures peuvent constituer respectivement un profilé de lisse destiné à être fixé sur une paroi murale pour former, avec une toile tendue, un faux-plafond ou bien un profilé de cadre. Les figures montrent un profilé de lisse en position de fixation sur une paroi murale.

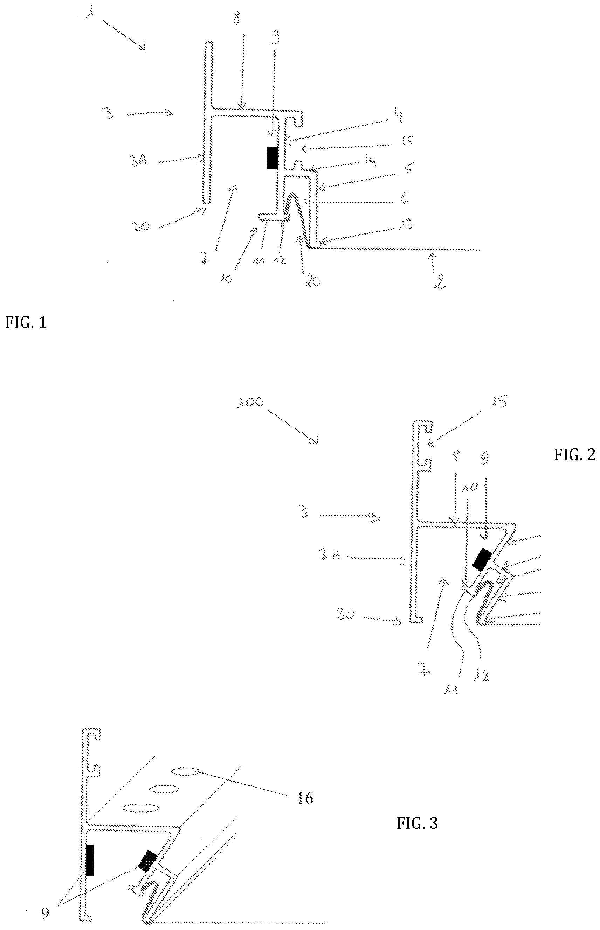

La figure 1 montre un premier exemple de réalisation d'un profilé de lisse 1 sur lequel une toile tendue est accrochée. Dans l'exemple illustré, le profilé de lisse 1 présente une première aile 3 verticale pour la fixation du profilé de lisse 1 sur une paroi (non représentée), une première gorge 6 arrangée pour permettre l'accroche de la toile 2 et une deuxième gorge 7 arrangée entre la première gorge 6 et la première aile 3.

Les première et deuxième gorges 6, 7 présentent respectivement une ouverture vers le bas. Elles sont délimitées latéralement l'une de l'autre par une paroi commune 4. Dans le mode de réalisation illustré, la paroi commune 4 s'étend parallèlement à la première aile 3. Le profilé de lisse 1 présente une deuxième aile 5 parallèle à la paroi commune 4. La première gorge 6 est ainsi délimitée latéralement par la deuxième aile 5 et une portion de la paroi commune 14. Dans le mode de réalisation illustré, la première aile 3, la paroi commune 4 et la deuxième aile 5 sont parallèles entre elles.

Le profilé de lisse peut être équipé d'une ou plusieurs cartouche(s) filtrante destinées à filtrer des odeurs en provenance de la pièce. Pour ce faire, la cartouche filtrante contient un matériau sorbant tels que de la zéolite. Il est bien entendu évident que le matériau sorbant sera choisi en fonction du type d'odeurs que l'on cherche à éliminer de la pièce. En complément du matériau sorbant, il peut être prévu que la cartouche filtrante contienne également une substance parfumée. Ladite cartouche sera arrangée alors pour assurer la libération de la substance parfumée. Ainsi, en complément de l'absorption ou adsorption de mauvaises odeurs, un composé volatil parfumé pourra être diffusé au sein de la pièce. ladite cartouche étant arrangée pour libérer ladite substance.

Dans le mode de réalisation illustré, la cartouche filtrante 9 est logée dans la deuxième gorge 7. Dans l'exemple donné, elle est fixée sur la paroi commune 4. Dans le cas de cartouche diffusant également des substances parfumées, la substance parfumée sera diffusée directement depuis la gorge du profilé de lisse 1 vers l'intérieur du local.

Afin de s'affranchir de l'aspect inesthétique de la cartouche 9, la paroi commune 4 est pourvue au niveau de son extrémité inférieure d'un épaulement 11 s'étendant vers l'intérieur de la deuxième gorge 7. L'épaulement est tel qu'il présente une longueur suffisante pour masquer la cartouche 9 aux yeux d'une personne placée dans le local, sous le faux-plafond.

Dans le mode de réalisation illustré, l'épaulement 11 est ménagé à l'extrémité inférieure de la paroi commune 4. Il présente en outre un prolongement 12 s'étendant vers l'intérieur de la première gorge 6, ledit prolongement 12 formant un épaulement pour l'accroche d'un harpon 20 comme illustré ou tout autre moyen équivalent équipant la bordure de la toile 2. L'épaulement 11 et le prolongement 12 s'étendent sensiblement perpendiculairement à la paroi commune 4.

La figure 2 illustre une variante de réalisation du profilé de lisse 1 précédemment décrit. Dans cette variante de réalisation, le profilé de lisse, référencé 100, diffère de celui précédemment décrit notamment par l'orientation de la paroi commune 4 et de la deuxième aile 5.

Plus particulièrement, la paroi commune 4 et la deuxième aile 3 sont agencées pour converger en direction de la portion inférieure 3A de la première aile 3. Cela a pour avantage d'améliorer le masquage de la cartouche filtrante 9 ainsi que celui des éléments d'accroche de la toile 2 sur la lisse 100 (harpon 20 ou moyens équivalents).

Avantageusement, la deuxième aile 5 présente une longueur telle que son extrémité libre (extrémité portant la patte d'appui de la toile) est située au même niveau que l'extrémité libre 30 de la portion inférieure 3A de la première aile. La paroi commune 4 est quant à elle dimensionnée pour présenter une extrémité s'arrêtant plus que l'extrémité inférieure 30 de la portion inférieure 3A de la première aile 3.

Avantageusement, la paroi commune 4 présente une longueur telle que le prolongement 12 et l'extrémité libre de la deuxième aile 5 soient alignés sensiblement verticalement. Cette configuration a pour avantage de rendre le harpon invisible à une personne placée sous le faux-plafond, et en particulier directement sous la lisse.

Par ailleurs, dans cette variante de réalisation, le rail 15 pour l'assemblage du profilé de lisse avec un autre profilé de lisse est porté par la portion de la première aile 3 située au-dessus de l'âme 8.

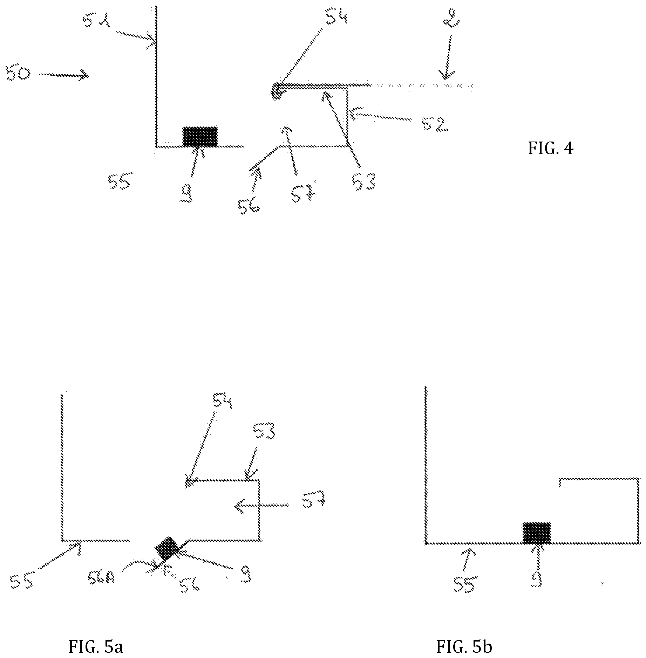

La figure 4 illustre un autre exemple de réalisation de profilé de lisse 50 constituant une structure de neutralisation d'odeur. Dans cet exemple de réalisation, le profilé de lisse 50 comprend une première et une deuxième ailes 51, 52 parallèles entre elles, l'une (ici l'aile 52) étant destinée à être fixée sur une paroi (non représentée). Le profilé de lisse 50 comporte en outre un bras 53 s'étendant perpendiculairement depuis l'aile 52 en direction de l'aile 51. Le bras 53 est pourvu en extrémité libre d'un épaulement 54 arrangé pour permettre l'accroche de la toile tendue 2 comme montré sur la figure 3. Les ailes 51, 52 sont reliées entre elles en partie inférieure par une paroi de raccordement 55 (également appelée paroi inférieure). Les ailes 51, 52 définissent avec la paroi inférieure 55 une gorge 57 débouchant dans l'espace situé au-dessus de la toile tendue 2 (vélum) lorsque celle-ci est accrochée sur le profilé de lisse 50. La cartouche filtrante 9 est située à l'intérieur de la gorge 57. Dans le mode de réalisation illustré, elle est disposée sur la paroi inférieure 55 qui constitue le fond de la gorge.

Afin de permettre le passage des émanations/composés volatiles depuis le local dans le vélum et le cas échéant la diffusion des substances volatiles parfumées au sein du local lorsque la cartouche filtrante combine la fonction de filtre et la fonction de diffuseur, la toile 2 mise en œuvre est avantageusement micro-perforée.

Afin de faciliter le remplacement de la cartouche filtrante 9, et en particulier éviter de décrocher la toile 2 pour procéder au retrait ou au remplacement de la cartouche, le profilé de lisse 50 comporte avantageusement un clapet d'accès 56. Dans le mode de réalisation illustré, le clapet d'accès 56 est ménagé au niveau de la paroi inférieure 55. Il est ainsi possible d'accéder facilement à l'intérieur de la gorge 57 et de procéder à la mise en place de la cartouche filtrante 9, à son retrait ou au remplacement de celle-ci sans avoir recours au décrochage de la toile 2. Afin d'améliorer le passage des odeurs à neutraliser présentes à l'intérieur du local ainsi que la diffusion de composés parfumés lorsque la cartouche filtrante 9 contient de tels composés, il peut être prévu que la paroi inférieure 9 et/ou le clapet d'accès 9 soi(en)t pourvu(e) d'orifices.

Les figures 5a et 5b illustrent une variante de réalisation de la figure 4, la figure 5a montrant le clapet d'accès 56 à la gorge 57 en position ouverte, la figure 5b montrant le clapet d'accès en position fermée. Dans ce mode de réalisation, la cartouche filtrante 9 est fixée sur le clapet d'accès 56, sur la face intérieure 56A de ce dernier. Par face intérieure du clapet, on entend la face orientée vers l'intérieur de la gorge lorsque le clapet d'accès 56 est en position fermée. La fixation de la cartouche filtrante 9 peut être fixée sur le clapet par collage ou tout autre moyen connu de l'homme du métier. Il peut être prévu également que la face intérieure 56A du clapet soit pourvue de moyens de retenu, tels que des ergots, délimitant un emplacement dans lequel la cartouche est placée et maintenue en force ou par clipsage.

L'avantage de l'arrangement des profilés de lisse avec la cartouche illustrés sur les figures 4 et 5a et 5b est de permettre la neutralisation des odeurs également présentes au sein de l'espace délimité par la face interne de la toile tendue (le vélum dans le cas d'un faux-plafond).

Avantageusement, il peut être prévu que le profilé comporte des ouvertures de passage 16 pour permettre le passage d'air entre le plénum (espace situé derrière la toile 2) et le local. La figure 3 montre, à titre d'exemple, le profilé de la figure 2 pourvu de telles ouvertures. Il est bien entendu évident que de telles ouvertures peuvent être prévus sur tout type de forme de profilé d'accroche selon l'invention. Dans l'exemple illustré, les ouvertures de passage d'air 16 sont portées par l'âme 8. On comprend cependant que l'emplacement de ces ouvertures sera défini selon la forme du profilé mais également de la position des cartouches filtrantes. Il est en effet avantageux de prévoir que les ouvertures de passage soient situées de manière à permettre le passage de l'air circulant entre le plénum et le local au travers de la gorge logeant une cartouche filtrante. Dans le mode de réalisation illustré, les ouvertures de passage 16 définissent des fenêtres de forme circulaire. Il s'agit bien entendu d'un exemple de réalisation, les ouvertures pouvant présenter une forme et/ou un agencement différent. Par ailleurs, le nombre d'ouverture est variable.

Dans les exemples précédemment décrits, les profilés d'accroche comprennent une seule cartouche. Il peut être prévu cependant que le profilé d'accroche comporte plusieurs cartouches, celles-ci pouvant être portée par une même paroi ou par des parois différentes (figure 3).

L'invention est décrite dans ce qui précède à titre d'exemple. Il est entendu que l'homme du métier est à même de réaliser différentes variantes de réalisation de l'invention sans pour autant sortir du cadre de l'invention tel que défini dans les revendications ci-jointes. En particulier, l'invention n'est pas limitée à l'arrangement ni aux profilés de lisse illustrés sur les figures précédemment décrites. Par ailleurs, il peut être prévu un profilé de lisse comprenant plusieurs cartouches filtrantes disposées dans une seule et même gorge ou dans des gorges distinctes.

## Revendications

1. Structure de neutralisation d'odeur(s) comprenant un profilé de lisse (1, 50, 100) adapté pour permettre l'accroche sur celui-ci d'une toile tendue (2) pour la réalisation d'une fausse-paroi et au moins une cartouche filtrante (9) contenant un matériau sorbant d'odeur(s) spécifiques, ladite cartouche étant logée dans une gorge (7, 57) du profilé de lisse.

2. Structure de neutralisation d'odeur(s) selon la revendication 1, **caractérisée en ce que** le matériau sorbant comprend de la zéolithe.

3. Structure de neutralisation d'odeur(s) selon la revendication 1 ou la revendication 2, **caractérisée en ce que** la cartouche filtrante (9) contient en outre une substance parfumée, ladite cartouche étant arrangée pour libérer ladite substance.

4. Structure de neutralisation d'odeur(s) selon l'une quelconque des revendications précédentes, **caractérisée en ce que** la gorge (7, 57) présente une ouverture débouchant au-dessus de la face interne de la toile qui est accrochée sur le profilé de lisse.

5. Structure de neutralisation d'odeur(s) selon l'une quelconque des revendications précédentes, **caractérisée en ce que** la gorge (7) présente une ouverture débouchant en partie inférieure du profilé de lisse.

6. Structure de neutralisation d'odeur(s) selon l'une quelconque des revendications précédentes, **caractérisée en ce que** la gorge (57) présente une ouverture débouchant dans un espace délimité par la face interne de ladite toile tendue (2) qui est accrochée sur le profilé de lisse.

7. Structure de neutralisation d'odeur(s) selon l'une quelconque des revendications précédentes, **caractérisée en ce que** le profilé de lisse (50) comporte un clapet d'accès (57) à la gorge (56).

8. Structure de neutralisation d'odeur(s) selon l'une quelconque des revendications précédentes, **caractérisée en ce que** la gorge (7, 57) est fermée par un cache pourvu de trous pour le passage de composés volatiles.

9. Structure de neutralisation d'odeur(s) selon l'un quelconque des revendications précédentes, **caractérisé en ce qu'**elle comprend une toile tendue (2) accrochée sur ledit au moins profilé de lisse, ladite structure de neutralisation formant une fausse paroi.

10. Structure de neutralisation d'odeur(s) selon la revendication précédente, caractérisé en ce la toile tendue (2) est micro-perforée.

11. Local comprenant la structure de neutralisation d'odeur(s) selon la revendication 9 ou la revendication 10, **caractérisée en ce que** la fausse-paroi est montée dans le local de telle sorte que la gorge (7, 57) dans laquelle la cartouche filtrante est fixée est une gorge accessible depuis l'intérieur du local.

## Patentansprüche

1. Geruchsneutralisierender Aufbau mit einer Profilleiste (1, 50, 100), die zum Befestigen eines gespannten Gewebes (2) für die Herstellung einer Zwischenwand geeignet ist, und mindestens einer Filterpatrone (9), die ein spezifisches geruchsabsorbierendes Material enthält, wobei die Patrone in einer Nut (7, 57) der Profilleiste sitzt.

2. Geruchsneutralisierender Aufbau nach Anspruch 1, **dadurch gekennzeichnet, dass** das geruchsabsorbierende Material Zeolith umfasst.

3. Geruchsneutralisierender Aufbau nach Anspruch 1 oder 2, **dadurch gekennzeichnet, dass** die Filterpatrone (9) darüber hinaus einen Duftstoff enthält, wobei die Patrone so angeordnet ist, dass dieser Stoff freigesetzt wird.

4. Geruchsneutralisierender Aufbau nach einem der vorhergehenden Ansprüche, **dadurch gekennzeichnet, dass** die Nut (7, 57) eine Öffnung aufweist, die oberhalb der Innenseite des an der Profilleiste befestigten Gewebes endet.

5. Geruchsneutralisierender Aufbau nach einem der vorhergehenden Ansprüche, **dadurch gekennzeichnet, dass** die Nut (7) eine Öffnung aufweist, die im unteren Teil der Profilleiste endet.

6. Geruchsneutralisierender Aufbau nach einem der vorhergehenden Ansprüche, **dadurch gekennzeichnet, dass** die Nut (57) eine Öffnung aufweist, die in einem Raum endet, der von der Innenseite des an der Profilleiste befestigten gespannten Gewebes (2) begrenzt wird.

7. Geruchsneutralisierender Aufbau nach einem der vorhergehenden Ansprüche, **dadurch gekennzeichnet, dass** die Profilleiste (50) eine Zugangsklappe (57) zur Nut (56) aufweist.

8. Geruchsneutralisierender Aufbau nach einem der vorhergehenden Ansprüche, **dadurch gekennzeichnet, dass** die Nut (7, 57) mit einer Abdeckung verschlossen ist, die mit Löchern für den Durchgang flüchtiger Verbindungen versehen ist.

9. Geruchsneutralisierender Aufbau nach einem der vorhergehenden Ansprüche, **dadurch gekennzeichnet, dass** er ein gespanntes Gewebe (2) umfasst, das an der mindestens einen Profilleiste befestigt ist, wobei der geruchsneutralisierende Aufbau eine Zwischenwand bildet.

10. Geruchsneutralisierender Aufbau nach dem vorhergehenden Anspruch, **dadurch gekennzeichnet, dass** das gespannte Gewebe (2) mikroperforiert ist.

11. Raum mit dem geruchsneutralisierender Aufbau nach Anspruch 9 oder Anspruch 10, **dadurch gekennzeichnet, dass** die Zwischenwand in dem Raum so montiert ist, dass die Nut (7, 57), in der die Filterpatrone befestigt ist, vom Rauminneren aus zugänglich ist.

## Claims

1. A structure to neutralise odours, comprising a rail section (1, 50, 100) arranged to allow the hanging, on the section, of a stretched fabric (2) in order to produce a false partition and at least one filter cartridge (9) containing a specific odour-sorbent material, said cartridge being housed in a groove (7, 57) of the rail section.

2. A structure to neutralise odours, according to claim 1, **characterised in that** the sorbent material comprises zeolite.

3. A structure to neutralise odours, according to claim 1 or claim 2, **characterised in that** the filter cartridge (9) further contains a fragrant substance, said cartridge being arranged to release said substance.

4. A structure to neutralise odours, according to any one of the preceding claims, **characterised in that** the groove (7, 57) has an aperture opening above the inner face of the fabric when the latter is attached to the rail section.

5. A structure to neutralise odours, according to any one of the preceding claims, **characterised in that** the groove (7) has an aperture opening in the lower part of the rail section.

6. A structure to neutralise odours, according to any one of the preceding claims, **characterised in that** the groove (57) has an aperture opening into a space delimited by the inner face of said stretched fabric (2) when the latter is attached to the rail section.

7. A structure to neutralise odours, according to any one of the preceding claims, **characterised in that** the rail section (50) has a flap (57) giving access to the groove (56).

8. A structure to neutralise odours, according to any one of the preceding claims, **characterised in that** the groove (7, 57) is closed by a cover provided with holes for the passage of volatile compounds.

9. A structure to neutralise odours, according to any one of the preceding claims, **characterised in that** it comprises a stretched fabric (2) hung on said at least one rail profile, said neutralisation structure forming a false partition.

10. A structure to neutralise odours, according to the previous claim, **characterised in that** the stretched fabric (2) is micro-perforated.

11. A room comprising the structure to neutralise odours, according to claim 9 or claim 10, **characterised in that** the false partition is mounted in the room such that the groove (7, 57) in which the filter cartridge is fixed is a groove accessible from inside the room.
